# EUROPEAN PATENT SPECIFICATION

(11) **EP 3 344 322 B1**
(45) Date of publication and mention of the grant of the patent: **25.12.2019**
(21) Application number: 16766156.0
(22) Date of filing: 31.08.2016
(51) Int. Cl.: A61M 25/00, A61L 29/08, A61M 1/28, A61M 5/162

(54) **IV ANTICOAGULANT TREATMENT SYSTEMS AND METHODS**
INTRAVENÖSE GERINNUNGSHEMMENDE BEHANDLUNGSSYSTEME UND -VERFAHREN
SYSTÈMES ET PROCÉDÉS DE TRAITEMENT ANTICOAGULANTS POUR PERFUSION INTRAVEINEUSE

(30) Priority: 03.09.2015 US 201562213920 P; 30.08.2016 US 201615251513
(43) Date of publication of application: 11.07.2018
(73) Proprietor: Becton, Dickinson and Company, Franklin Lakes, NJ 07417-1880 (US)
(72) Inventor: MA, Yiping, Layton, Utah 84040 (US); TAI, Jeff, Lehi, Utah 84043 (US)
(74) Representative: dompatent von Kreisler Selting Werner - Partnerschaft von Patent- und Rechtsanwälten mbB
(86) International application number: PCT/US2016/049698
(87) International publication number: WO 2017/040661

(56) References cited:
- EP-A1- 0 747 073
- WO-A1-91/08790
- US-A1- 2013 158 517
- US-A1- 2014 114 260
- US-A1- 2015 133 846
- YOON KI JOUNG ET AL: "Heparin-Conjugated Pluronic Nanogels as Multi-Drug Nanocarriers for Combination Chemotherapy", MOLECULAR PHARMACEUTICS, vol. 10, no. 2, 4 February 2013 (2013-02-04), pages 685-693, XP055320594, US ISSN: 1543-8384, DOI: 10.1021/mp300480v

## Description

### BACKGROUND

The present invention is directed to
an intravenous ("IV") delivery system, by which fluids can be administered directly to a patient as well as a method for manufacturing
an intravenous delivery system. An intravenous delivery system according to the invention is used broadly herein to describe components used to deliver the fluid to the patient, for use in arterial, intravenous, intravascular, peritoneal, and/or non-vascular administration of fluid. Of course, one of skill in the art may use an intravenous delivery system to administer fluids to other locations within a patient's body.

One common method of administering fluids into a patient's blood flow is through an intravenous delivery system. In many common implementations, an intravenous delivery system may include a liquid source such as a liquid bag, a drip chamber used to determine the flow rate of fluid from the liquid bag, tubing for providing a connection between the liquid bag and the patient, and an intravenous access unit, such as a catheter that may be positioned intravenously in a patient. An intravenous delivery system may also include a Y-connector that allows for the piggybacking of intravenous delivery systems and for the administration of medicine from a syringe into the tubing of the intravenous delivery system.

Known catheter designs are subject to occlusion due to blood clot formation. Such occlusions may necessitate premature replacement of catheter components, requiring time and attention from health care professionals. Such occlusions are typically caused by the formation of a blood clot on the catheter surfaces, which eventually grows to a size sufficient to block fluid flow. In some instances, the clot may be flushed out of the catheter if flushing is carried out on a regular basis. In other cases, flushing may not remove the clots. Accordingly, conventional catheter flushing processes are not sufficiently reliable.

US 2013/158517 relates to catheters for introduction and removal of fluids from the human body according to the preamble of claim 1, wherein the catheters comprise a polymeric material on the exterior and/or intraluminal surfaces thereof to reduce microbial attachment, biofilm formation, platelet attachment or thrombus formation. Also disclosed is a process for the preparation of said catheter according to the preamble of claim 8.

### BRIEF SUMMARY OF THE INVENTION

Embodiments of the present invention arc generally directed to intravenous delivery systems that provide enhanced resistance to blood clot formation, and to methods for manufacturing such intravenous delivery systems.

Reference(s) to "embodiment(s)" throughout the description which; are not under the scope of the appended claims merely represent possible exemplary executions and are therefore not part of the present invention.

The intravenous delivery system comprises a plurality of components that have a plurality of interior surfaces that cooperate to define a fluid pathway through which medication flows into a body of a patient. The intravenous delivery system also has one or more anticoagulant coatings on at least a first interior surface of the plurality of interior surfaces. The one or more anticoagulant coatings restricts blood clot formation in the fluid pathway.

The one or more anticoagulant coatings includes a triblock copolymer. The triblock copolymer may be covalently bonded to the first interior surface. The triblock copolymer may be one of PEO-PPO-PEO and PEO-PBD-PEO. More specifically, the triblock copolymer may be designated by the trade name Pluronic® F108, from BASF Corporation. The one or more anticoagulant coatings may be attached to the first interior surface as one or more PEO brush layers, each having a thickness of less than 20 nanometers.

The first interior surface may be on a first component of the plurality of components. The first component may be a catheter tubing tip, catheter tubing, a catheter adapter, integrated extension tubing, or a Luer connect port.

The plurality of components may further have a plurality of exterior surfaces. The one or more anticoagulant coatings may be on substantially all of the plurality of interior surfaces, and on a first exterior surface of the plurality of exterior surfaces.

According to the method fo the invention an intravenous delivery system is manufactured. The method includes providing a plurality of components of the intravenous delivery system such that the plurality of components have a plurality of interior surfaces that cooperate to define a fluid pathway through which fluid flows into a body of a patient. The method further includes preparing an anticoagulant solution and exposing at least a first interior surface of the plurality of interior surfaces to the anticoagulant solution to form one or more anticoagulant coatings that restrict blood clot formation in the fluid pathway. The method further includes causing the one or more anticoagulant coatings to adhere to at least the first interior surface.

Preparing the anticoagulant solution includes dissolving a triblock copolymer in water. The triblock copolymer may be PEO-PPO-PEO or PEO-PBD-PEO. Preparing the anticoagulant solution dissolving Nisin and/or low molecular weight heparin in the water.

Causing the one or more anticoagulant coatings to adhere to at least the first interior surface may include forming a covalent bond between the one or more anticoagulant coatings and the first interior surface. Forming the covalent bond may include applying radiation to the one or more anticoagulant coatings and the first interior surface to induce formation of the covalent bond. Applying radiation to the one or more anticoagulant coatings and the first interior surface may include applying gamma irradiation, ultraviolet irradiation and/or electron beam irradiation to the one or more anticoagulant coatings and the first interior surface.

Exposing at least a first interior surface of the plurality of interior surfaces to the anticoagulant solution may include attaching, the one or more anticoagulant coatings to the first interior surface as one or more PEO brush layers. Each of the PEO brush layers may have a thickness of less than 20 nanometers. Further, exposing at least a first interior surface of the plurality of interior surfaces to the anticoagulant solution may include exposing, to the anticoagulant solution, a first component of the plurality of components, which may be a catheter tubing tip, catheter tubing, a catheter adapter, integrated extension tubing, or a Luer connect port.

Exposing at least a first interior surface of the plurality of interior surfaces to the anticoagulant solution may include exposing substantially all of the plurality of interior surfaces to the anticoagulant solution. The plurality of components may further include a plurality of exterior surfaces. The method may further include exposing a first exterior surface of the plurality of exterior surfaces to the anticoagulant solution.

The method for manufacturing an intravenous delivery system includes providing a plurality of components of the
intravenous delivery system such that the plurality of components have a plurality of interior surfaces that cooperate to define a fluid pathway through which fluid flows into a body of a patient. The plurality of components may include at least catheter tubing, an adapter, and integrated tubing. The method may further include preparing an anticoagulant solution, and exposing at least a subset of interior surfaces of the plurality of interior surfaces to the anticoagulant solution to form anticoagulant coatings on the subset of interior surfaces that restrict blood clot formation in the fluid pathway. The subset of interior surfaces may be on at least the catheter tubing, the adapter, and the integrated tubing. The method may further include applying radiation to the anticoagulant coatings and the subset of interior surfaces to form covalent bonds between the anticoagulant coatings and the subset of interior surfaces.

Preparing the anticoagulant solution includes dissolving a triblock copolymer in water or other solutions such as saline. The triblock copolymer may be designated by the trade name Pluronic® F108, from BASF Corporation.

### BRIEF DESCRIPTION OF THE SEVERAL VIEWS OF THE DRAWINGS

In order that the manner in which the above-recited and other features and advantages of the invention are obtained will be readily understood, a more particular description of the invention briefly described above will be rendered by reference to specific embodiments thereof that are illustrated in the appended drawings. These drawings depict only typical embodiments of the invention and are not therefore to be considered to limit the scope of the invention.
Figure 1 is a plan view of an intravenous delivery system according to one embodiment;
Figure 2 is a flowchart diagram illustrating a method of manufacturing the intravenous delivery system of Figure 1, according to one embodiment;
Figure 3 is a cross-sectional view of the intravenous delivery system, according to embodiments;
Figure 4 is an enlarged cross-sectional view of a portion of the intravenous delivery system, according to some embodiments; and
Figure 5 is an enlarged cross-sectional view of another portion of the intravenous delivery system, according to some embodiments.

### DETAILED DESCRIPTION OF THE INVENTION

The presently preferred embodiments of the present invention can be understood by reference to the drawings, wherein like reference numbers indicate identical or functionally similar elements.

Moreover, the Figures may show simplified or partial views, and the dimensions of elements in the Figures may be exaggerated or otherwise not in proportion for clarity. In addition, the singular forms "a," "an," and "the" include plural referents unless the context clearly dictates otherwise. Thus, for example, reference to a terminal includes reference to one or more terminals. In addition, where reference is made to a list of elements (e.g., elements a, b, c), such reference is intended to include any one of the listed elements by itself, any combination of less than all of the listed elements, and/or a combination of all of the listed elements.

The term "substantially" means that the recited characteristic, parameter, or value need not be achieved exactly, but that deviations or variations, including for example, tolerances, measurement error, measurement accuracy limitations and other factors known to those of skill in the art, may occur in amounts that do not preclude the effect the characteristic was intended to provide.

As used herein, the term "proximal", "top", "up" or "upwardly" refers to a location on the device that is closest to the clinician using the device and farthest from the patient in connection with whom the device is used when the device is used in its normal operation. Conversely, the term "distal", "bottom", "down" or "downwardly" refers to a location on the device that is farthest from the clinician using the device and closest to the patient in connection with whom the device is used when the device is used in its normal operation.

As used herein, the term "in" or "inwardly" refers to a location with respect to the device that, during normal use, is toward the inside of the device. Conversely, as used herein, the term "out" or "outwardly" refers to a location with respect to the device that, during normal use, is toward the outside of the device.

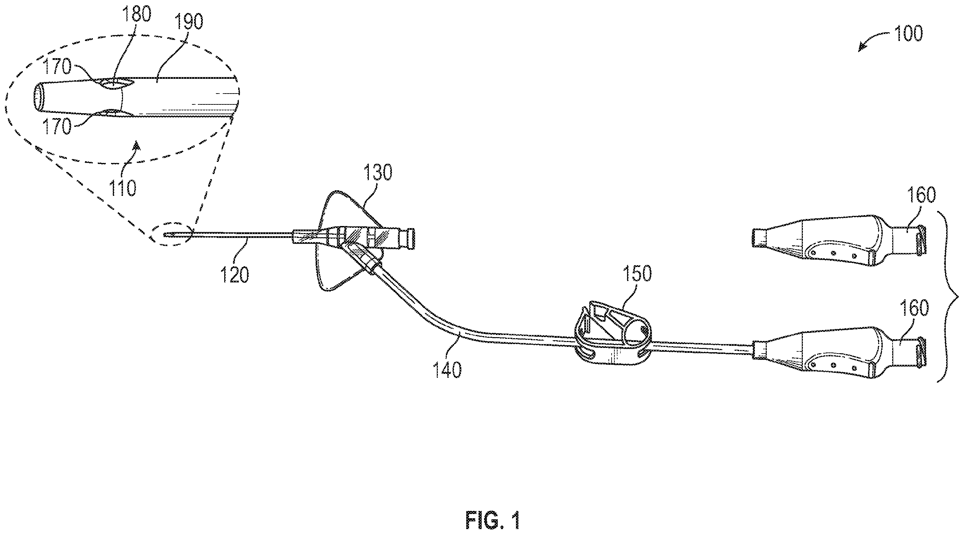

Referring to Figure 1, a plan view illustrates an intravenous delivery system 100 according to one embodiment. The intravenous delivery system 100 may have a plurality of components that convey a fluid, such as medication or blood, to the body of a patient. The intravenous delivery system 100 may include various components, some of which are shown in Figure 1, by way of example. As shown, the intravenous delivery system 100 may include a catheter tubing tip 110, catheter tubing 120, a catheter adapter 130, extension tubing 140, a clip 150, and a Luer connect port 160.

The Luer connect port 160 may be used to connect the intravenous delivery system 100 to a fluid source such as an IV bag or drip chamber (not shown). The clip 150 may be used to selectively reduce or stop fluid flow through the extension tubing 140 by compressing the extension tubing 140. The clip 150 may be selectively pressed into a clamping state, or released from the clamping state, by a user. The catheter adapter 130 may be used to facilitate introduction of another fluid into the intravenous delivery system 100, to be delivered to the patient along with the fluid flowing through the extension tubing 140. The catheter tubing 120 may be inserted through the patient's skin into the part of the body into which the fluid is to be administered, for example, into a blood vessel. The catheter tubing tip 110, which may be a tapered and sharpened tip of the catheter tubing 120, may be used for penetration of the tissue to access the fluid delivery site, and may reside in the fluid delivery site during delivery of the fluid to the patient.

As shown, the catheter tubing tip 110 may have a plurality of diffuser holes 170, which may enable the fluid to flow from the catheter tubing tip 110 in multiple directions, thereby diffusing fluid flow. The catheter tubing tip 110 may have an interior surface 180, which can be seen through the diffuser holes 170 and helps define a fluid pathway through the catheter tubing tip 110. Further, the catheter tubing tip 110 may have an exterior surface 190, which faces outward and contacts the tissue of the patient during introduction of the catheter tubing tip 110 into the fluid delivery site and remains in contact with the tissue during delivery of the fluid.

Like the catheter tubing tip 110, each of the other components of the intravenous delivery system 100, with the exception of the clip 150 (i.e., the catheter tubing 120, the catheter adapter 130, the extension tubing 140, and the Luer connect port 160) may have an interior surface and an exterior surface. The various interior surfaces of the fluid-conveying components of the intravenous delivery system 100 (the catheter tubing tip 110, the catheter tubing 120, the catheter adapter 130, the extension tubing 140, and the Luer connect port 160) cooperate to define a fluid pathway through which fluid flows through the intravenous delivery system 100, into the body of the patient.

These interior surfaces may be in contact with blood and/or other fluids, such as the fluid to be administered to the patient, which may potentially cause blood clot formation. Further, some of the exterior surfaces, such as the exterior surface 190 of the catheter tubing tip 110 and the corresponding exterior surface of the catheter tubing 120, may be in contact with blood and/or other fluids within the body of the patient. Accordingly, these interior and exterior surfaces are locations at which blood clots may adhere and grow. Such blood clots may occlude blood flow.

Accordingly, it may be desirable to provide an anticoagulant coating on some or all of these interior and exterior surfaces. In some aspects of the description, all of the interior surfaces
and exterior surfaces of all components of the intravenous delivery system 100 may have an anticoagulant coating. In other aspects of the description only the interior and exterior surfaces of the
components of the intravenous delivery system 100 that convey fluid (the catheter tubing tip 110, the catheter tubing 120, the catheter adapter 130, the extension tubing 140, and the Luer connect port 160) may have the anticoagulant coating.

In yet other embodiments, all of the interior surfaces and only some of the exterior surfaces of the fluid-conveying components of the intravenous delivery system 100 have the anticoagulant coating. Only the exterior surfaces expected to contact bodily or delivered fluid may be coated. For example, along with the interior surfaces, the exterior surface 190 of the catheter tubing tip 110 and the corresponding exterior surface of the catheter tubing 120 may have the anticoagulant coating.

In still other embodiments, all of the interior surfaces, and none of the exterior surfaces, of the fluid-conveying components of the intravenous delivery system 100 may have the anticoagulant coating. In yet other embodiments, only some of the interior surfaces of the fluid-conveying components of the intravenous delivery system 100 may have the anticoagulant coating. In still other embodiments, only one interior surface of one fluid-conveying component of the intravenous delivery system 100 may have the anticoagulant coating. For example, only the interior surface 180 of the catheter tubing tip 110 may have the anticoagulant coating. In embodiments in which not all interior surfaces have the anticoagulant coating, only the interior surface(s) deemed to be at greatest risk for blood clot formation and/or occlusion may be coated.

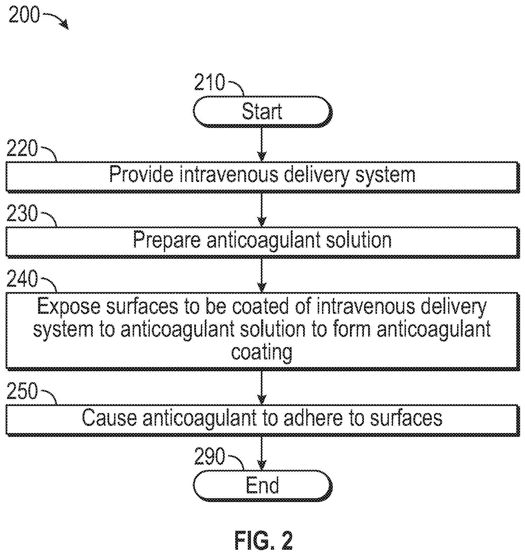

The intravenous delivery system 100 is merely exemplary. The anticoagulant coating may be formed in a wide variety of ways. Some exemplary manufacturing methods will be shown and described in connection with Figure 2.

Figure 2 is a flowchart diagram illustrating a method 200 of manufacturing an intravenous delivery system according to one embodiment. The method 200 will be described in conjunction with the intravenous delivery system 100 of Figure 1, as though used to manufacture the intravenous delivery system 100. However, those of skill in the art will recognize that the method 200 may be used to manufacture a wide variety of intravenous delivery system besides the intravenous delivery system 100 of Figure 1, within the scope of the present disclosure. Similarly, the intravenous delivery system 100 of Figure 1 may be made through the use of a variety of other methods, aside from the method 200 of Figure 2, within the scope of the present disclosure.

The method 200 starts 210 with a step 220 in which the intravenous delivery system 100 is provided. The various components of the intravenous delivery system 100 (or other components, in the event that the method 200 is used to manufacture a different intravenous delivery system) may be manufactured through the use of any methods known in the art. The components of the method 200 may optionally be coupled together (for example, in the manner illustrated in Figure 1) prior to undertaking further steps.

In a step 230, an anticoagulant solution is provided. The anticoagulant solution is prepared by inter alia dissolving a triblock copolymer in water or other solvents. A variety of
anticoagulants may be used. In some embodiments, the anticoagulant may be a triblock copolymer. Some exemplary triblock copolymers include PEO-PPO-PEO and PEO-PBD-PEO, where PEO is polyethylene oxide, PPO is polypropylene oxide, and PBD is polybutadiene. More specifically, the triblock copolymer may be of a type sold under the name of Pluronic®, marketed by BASF Corporation. Yet more specifically, the triblock copolymer may include Pluronic® F 108, Pluronic® F 68, and/or Pluronic® F 127. In some embodiments, an end-activated group Pluronic® (E.G.A.P.) may be used.

These and other anticoagulants that may be used within the scope of the present disclosure may adhere to the surfaces to be coated via adsorption, and may "self-arrange" on the surfaces to be coated. For example, in the case of the triblock copolymers mentioned above, the PEO component of these molecules may be hydrophilic, while the central molecule (PPO or PBD) may be hydrophobic. The PPO or PBD domains, as hydrophobic molecules, may self-arrange on the surfaces to be coated in response to contact of the anticoagulant solution with the surfaces to be coated. The PEO domains, as hydrophilic molecules, may point away from the surfaces to be coated, thereby forming a "PEO brush layer." The presence of the PEO brush layer may inhibit adsorption of (serum) proteins and/or aggregation of platelets on the interior surfaces that have been coated, thereby delaying and/or eliminating blood clot formation on those surfaces.

Various concentrations of the triblock copolymer may be dissolved in the water. In some embodiments, the concentration of the triblock copolymer may range from 1 mg / mL of water to 20 mg / mL of water. More specifically, the concentration of the triblock copolymer may range from 2 mg / mL of water to 10 mg / mL of water. Yet more specifically, the concentration of the triblock copolymer may range from 3 mg / mL of water to 7 mg / mL of water. Still more specifically, the concentration of the triblock copolymer may be 5 mg / mL of water.

The actual concentration of anticoagulant in the anticoagulant solution may be dependent upon the manner in which the anticoagulant is to be applied to the surfaces to be coated in subsequent steps, the surface area of these surfaces, the particular type of anticoagulant used, and/or other factors. Thus, the concentration of anticoagulant in the anticoagulant solution may be tuned to the specific manufacturing process. The key may be to ensure that a sufficient quantity of anticoagulant is present in the anticoagulant solution to coat all surfaces to be coated with the desired coverage area. It may be acceptable to use a higher concentration of the anticoagulant because any suspended molecules that remain after adherence of the anticoagulant to the surfaces to be coated may be eluted away from the coated surfaces.

In some embodiments, the anticoagulant solution may be applied so as to provide a very thin PEO brush layer, for example, ranging in thickness from 1 nm to 20 nm in thickness. More specifically, the PEO brush layer may range in thickness from 5 nm to 15 nm in thickness. Yet more specifically, the PEO brush layer may range in thickness from 8 nm to 12 nm in thickness. Still more specifically, the PEO brush layer may be 10 nm in thickness.

In some embodiments, the anticoagulant solution may also include an anticoagulant additive to enhance the anticoagulant properties. Many different anticoagulant additives may be used within the scope of the present disclosure. One example is low molecular weight heparin (LMWH). The anticoagulant additive may be dissolved in water or other solutions after the formation of the tri-block copolymer coating on the device surface. The anticoagulant additive will then be entrapped in the triblock copolymer brush layer. Various concentrations of LMWH may be used. As with the triblock copolymer, the concentration of the anticoagulant additive in the anticoagulant solution may be tuned to the specific manufacturing process, with the possibility of eluting away excess suspended molecules.

The anticoagulant and/or the anticoagulant additive, as applicable, is dissolved in water or other solution according to any known procedure to form the anticoagulant solution. The step 230 is then complete.

Once the anticoagulant solution has been prepared, the method 200 proceeds to a step 240 in which the surfaces of the intravenous delivery system 100 to be coated are exposed to the anticoagulant solution. This may be done in a wide variety of ways.

According to one method, a "fill and drain" method may be used. The intravenous delivery system 100 may be filled with the anticoagulant solution, for example, through the use of a syringe containing the anticoagulant solution. The tip of the syringe may be inserted into one of the open ends of the intravenous delivery system 100 (for example, the end of the catheter tubing tip 110 or the end of the Luer connect port 160). The other end of the intravenous delivery system 100 may be left open so that the anticoagulant solution passes through the intravenous delivery system 100 and exits the intravenous delivery system 100 through the open end. Alternatively, the other end of the intravenous delivery system 100 may be plugged so that the intravenous delivery system 100 is more likely to fill with the anticoagulant solution, thereby providing more complete exposure of the interior surfaces of the intravenous delivery system 100 to the anticoagulant solution.

The intravenous delivery system 100 may remain plugged until the anticoagulant solution has remained in contact with the surfaces to be coated for a predetermined length of time. If desired, the syringe may be removed, and the end to which it was coupled may also be plugged for convenience so that the intravenous delivery system 100 can easily be left in place while the anticoagulant adheres to the surfaces to be coated. The length of time needed may depend on the particular components of the anticoagulant solution, the surface area of the surfaces to be coated, the concentration of the various solutes in the anticoagulant solution, the ambient temperature, the hydrophobicity of the surface, and/or other factors.

In some embodiments, the anticoagulant may adsorb to the surfaces to be coated substantially immediately, requiring no significant resting time. In other embodiments, the anticoagulant solution may be left in contact with the surfaces to be coated for a few minutes, a few hours, or even a few days in order to provide sufficient time for the anticoagulant molecules to auto-arrange on and adhere to the surfaces to be coated. In some exemplary embodiments, the anticoagulant solution may be left to incubate for about four hours at room temperature (23°C) to allow the auto-arrangement and adherence to occur.

As mentioned previously, it may be desirable to coat one or more of the exterior surfaces of the intravenous delivery system 100 in addition to one or more of the interior surfaces. In order to accomplish this, alternative exposure methods may be used. According to one alternative embodiment, the intravenous delivery system 100 may be dipped in the anticoagulant solution. The intravenous delivery system 100 may be dipped in its entirety in the anticoagulant solution; alternatively, only components of the intravenous delivery system 100 for which the interior and exterior surfaces are to be coated may be dipped. The intravenous delivery system 100 (or portions thereof) may remain immersed in the anticoagulant solution for the optimal period of time for adherence and auto-arrangement of the anticoagulant, as described previously.

These exposure methods are merely exemplary. Those of skill in the art will recognize that any known method whereby a surface can be exposed to the solute of a solution may be used to expose the surfaces of the intravenous delivery system 100 to be coated to the anticoagulant, and/or the anticoagulant additive. One exemplary alternative method is to spray the anticoagulant solution onto the surfaces to be coated. The spray may be a fine mist so as to atomize the anticoagulant solution, thereby providing relatively rapid and even coverage of the surfaces.

Once exposure is complete, the intravenous delivery system 100 may be removed from the anticoagulant solution and allowed to dry. As indicated previously, any excess suspended molecules (for example, the anticoagulant, the antibacterial additive, and/or the anticoagulant additive) may be eluted away from the surfaces to be coated.

The surfaces to be coated may now each have an anticoagulant coating formed by adherence and self-arrangement of the anticoagulant on the surfaces. The adherence of the anticoagulant coatings to the surfaces may be sufficient to prevent and/or resist blood clot formation during usage of the catheter. However, in some embodiments, it may be desirable to more securely bond the anticoagulant coatings to the surfaces to help the anticoagulant coatings to remain in place and/or extend the useful life of the anticoagulant coatings.

Accordingly, once the anticoagulant coatings have been formed on the surfaces to be coated, the method 200 may optionally proceed to a step 250 in which the anticoagulant coatings are caused to adhere to the surfaces that have been coated. This may be done in a variety of ways. According to some exemplary embodiments, covalent bonds may be formed between the triblock copolymers and the surfaces on which they reside. This may be done, according to some embodiments, by applying irradiation to the anticoagulant coatings and the surfaces on which they reside.

Irradiation may be applied according to a wide variety of procedures. Exemplary procedures include, but are not limited to, gamma irradiation, ultraviolet irradiation, and electron beam irradiation. Irradiation may be conducted for a time sufficient to cause the covalent bonds to form between the triblock copolymers and the surfaces to which they are applied. Irradiation may be conducted for a few minutes, a few hours, or even a few days in order to provide sufficient time for the covalent bonds to form. According to one example, the surfaces and anticoagulant coatings may be irradiated by a 60Co source over eight days to a total dose of 80 kGy.

In the alternative to application of irradiation, any other known method may be used to form the covalent bonds. Such alternatives may include the addition of binders and/or other agents in the anticoagulant solution to cause or facilitate formation of the covalent bonds. Further, in other alternatives, other methods, such as application of thermal energy, may be used to strengthen adherence of the anticoagulant layers to the surfaces through the use of one or more mechanisms besides covalent bonding.

Once the anticoagulant coatings have been caused to adhere to the surfaces with sufficient strength, the surfaces and anticoagulant coatings may be rinsed with water or other rinsing agents to remove any loosely-bound triblock copolymers. The method 200 may then end 290. The intravenous delivery system 100 may be ready for use. The anticoagulant coatings may prevent or delay blood clot formation within the fluid path defined by the interior surfaces of the intravenous delivery system 100 and/or on the exterior of the intravenous delivery system 100, depending on where the anticoagulant coating has been applied.

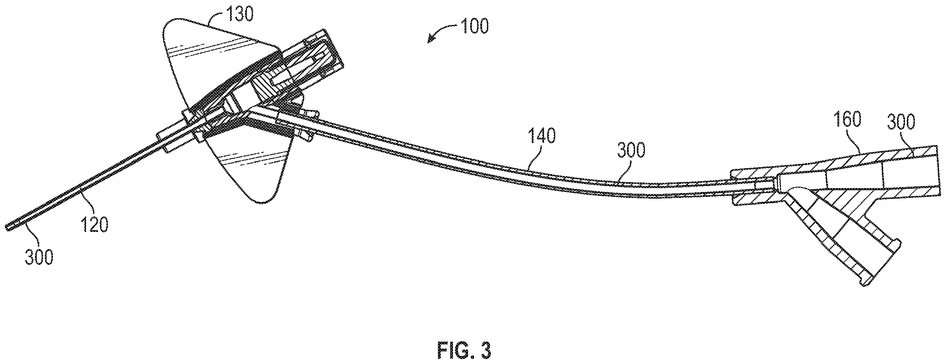

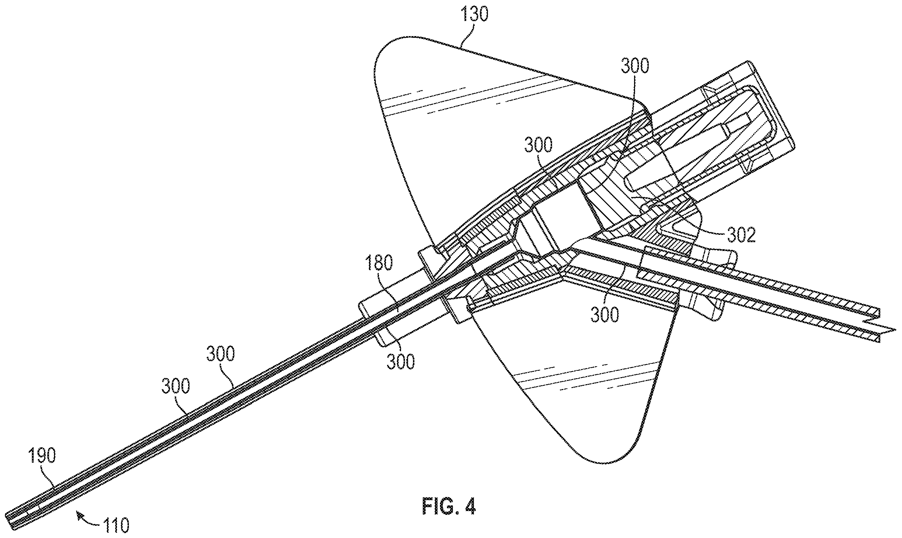

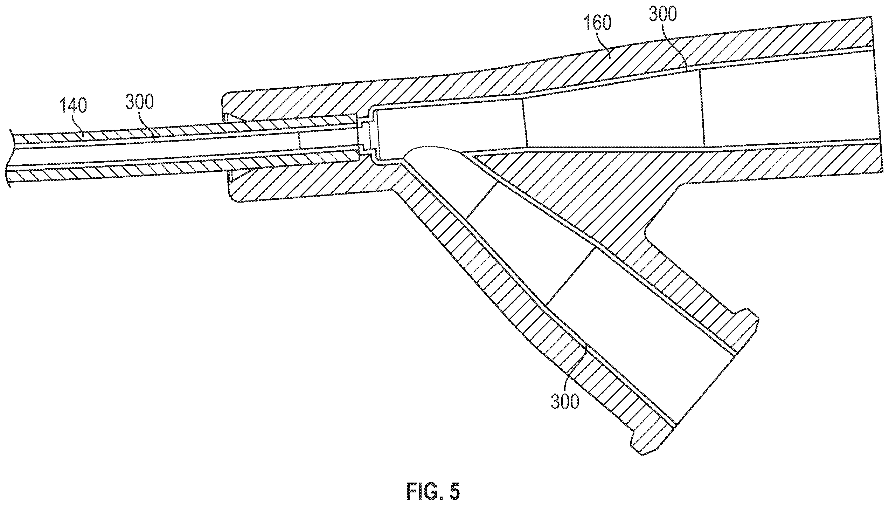

Figures 3-5 illustrate an anticoagulant coating 300 on a plurality of internal surfaces and at least one external surface of the intravenous delivery system 100. In some embodiments, the anticoagulant coating 300 may be applied to a distal surface of a septum 302.

## Claims

1. An intravenous delivery system (100) comprising:
a plurality of components comprising a plurality of interior surfaces that cooperate to define a fluid pathway through which fluid flows into a body of a patient; and
one or more anticoagulant coatings (300) on at least a first interior surface of the plurality of interior surfaces,
wherein the one or more anticoagulant coatings (300) restrict blood clot formation in the fluid pathway,
wherein the intravenous delivery system (100) is **characterized in that** the one or more anticoagulant coatings (300) comprise a triblock copolymer.

2. The intravenous delivery system (100) of claim 1, wherein the triblock copolymer is selected from the group consisting of PEO-PPO-PEO and PEO-PBD-PEO.

3. The intravenous delivery system (100) of claim 1, wherein the one or more anticoagulant coatings are covalently bonded to the first interior surface.

4. The intravenous delivery system (100) of any one of claims 1 to 3, wherein the one or more anticoagulant coatings (300) are attached to the first interior surface as one or more PEO brush layers, each having a thickness of less than 20 nanometers.

5. The intravenous delivery system (100) of claim 1, wherein the one or more anticoagulant coatings (300) further comprise low molecular weight heparin.

6. The intravenous delivery system (100) of any one of claims 1 to 5, wherein the first interior surface is on a first component of the plurality of components, wherein the first component is selected from the group consisting of:
a catheter tubing tip (110);
catheter tubing (120);
a catheter adapter (130);
integrated extension tubing (140); and
a Luer connect port (160).

7. The intravenous delivery system (100) of any one of claims 1 to 6, wherein the plurality of components further comprise a plurality of exterior surfaces, wherein the one or more anticoagulant coatings (300) are on substantially all of the plurality of interior surfaces, and on a first exterior surface of the plurality of exterior surfaces.

8. A method for manufacturing an intravenous delivery system (100), the method comprising:
providing a plurality of components of the intravenous delivery system (100) such that the plurality of components comprise a plurality of interior surfaces that cooperate to define a fluid pathway through which fluid flows into a body of a patient;
preparing an anticoagulant solution;
exposing at least a first interior surface of the plurality of interior surfaces to the anticoagulant solution to form a anticoagulant coatings (300) that restrict blood clot formation in the fluid pathway; and
causing the anticoagulant coatings (300) to adhere to at least the first interior surface;
wherein the method is **characterized in that** preparing an anticoagulant solution includes dissolving a triblock copolymer in water or other solutions.

9. The method of claim 8, wherein the triblock copolymer is selected from the group consisting of PEO-PPO-PEO and PEO-PBD-PEO.

10. The method of claim 9, wherein preparing the anticoagulant solution further comprises dissolving low molecular weight heparin or other anticoagulant drug molecule in the water or other suitable solution.

11. The method of any one of claims 8 to 10, wherein causing the anticoagulant coatings (300) to adhere to at least the first interior surface comprises forming a covalent bond between the anticoagulant coatings (300) and the first interior surface, preferably
wherein forming a covalent bond between the anticoagulant coating (300) and the first interior surface comprises applying radiation to the one or more anticoagulant coatings (300) and the first interior surface to induce formation of the covalent bond, more preferably
wherein applying radiation to the one or more anticoagulant coatings (300) and the first interior surface comprises applying a selection from the group consisting of gamma irradiation, ultraviolet irradiation and electron beam irradiation to the one or more anticoagulant coatings (300) and the first interior surface.

12. The method of any one of claims 8 to 11, wherein exposing at least a first interior surface of the plurality of interior surfaces to the anticoagulant solution comprises attaching the one or more anticoagulant coatings (300) to the first interior surface as one or more PEO brush layers, each having a thickness of less than 20 nanometers.

13. The method of claim 12, wherein exposing at least a first interior surface of the plurality of interior surfaces to the anticoagulant solution comprises exposing, to the anticoagulant solution, a first component of the plurality of components selected from the group consisting of:
a catheter tubing tip (110);
catheter tubing (120);
a catheter adapter (130);
integrated extension tubing (140); and
a Luer connect port (160).

14. The method of any one of claims 8 to 13, wherein exposing at least a first interior surface of the plurality of interior surfaces to the anticoagulant solution comprises exposing substantially all of the plurality of interior surfaces to the anticoagulant solution; and
wherein the plurality of components further comprise a plurality of exterior surfaces, the method further comprising exposing a first exterior surface of the plurality of exterior surfaces to the anticoagulant solution.

15. The method of claim 8, wherein the plurality of components comprises at least catheter tubing, an adapter, and integrated tubing;
exposing at least a subset of interior surfaces of the plurality of interior surfaces to the anticoagulant solution to form anticoagulant coatings (300) on the subset of interior surfaces that restrict blood clot formation in the fluid pathway, wherein the subset of interior surfaces is on at least the catheter tubing, the adapter, and the integrated tubing; and
applying radiation to the anticoagulant coatings (300) and the subset of interior surfaces to form covalent bonds between the anticoagulant coatings (300) and the subset of interior surfaces.

## Patentansprüche

1. Intravenöses Abgabesystem (100), umfassend:
eine Vielzahl von Komponenten, die eine Vielzahl von Innenflächen umfassen, welche zusammen einen Fluidweg definieren, durch den ein Fluid in einen Körper eines Patienten fließt; und
eine oder mehrere Antikoagulansbeschichtungen (300) auf wenigstens einer ersten Innenfläche der Vielzahl von Innenflächen;
wobei die eine oder die mehreren Antikoagulansbeschichtungen (300) die Blutgerinnselbildung in dem Fluidweg einschränken;
wobei das intravenöse Abgabesystem (100) **dadurch gekennzeichnet ist, dass** die eine oder die mehreren Antikoagulansbeschichtungen (300) ein Triblock-Copolymer umfassen.

2. Intravenöses Abgabesystem (100) gemäß Anspruch 1, wobei das Triblock-Copolymer aus der Gruppe ausgewählt ist, die aus PEO-PPO-PEO und PEO-PBD-PEO besteht.

3. Intravenöses Abgabesystem (100) gemäß Anspruch 1, wobei die eine oder die mehreren Antikoagulansbeschichtungen kovalent an die erste Innenfläche gebunden sind.

4. Intravenöses Abgabesystem (100) gemäß einem der Ansprüche 1 bis 3, wobei die eine oder die mehreren Antikoagulansbeschichtungen (300) als eine oder mehrere PEO-Bürstenschichten, die jeweils eine Dicke von weniger als 20 Nanometer aufweisen, an der ersten Innenfläche befestigt sind.

5. Intravenöses Abgabesystem (100) gemäß Anspruch 1, wobei die eine oder die mehreren Antikoagulansbeschichtungen (300) weiterhin niedermolekulares Heparin umfassen.

6. Intravenöses Abgabesystem (100) gemäß einem der Ansprüche 1 bis 5, wobei sich die erste Innenfläche auf einer ersten Komponente der Vielzahl von Komponenten befindet, wobei die erste Komponente aus der Gruppe ausgewählt ist, die aus Folgenden besteht:
einer Katheterschlauchspitze (110);
einem Katheterschlauch (120);
einem Katheteradapter (130);
integrierten Verlängerungsschläuchen (140); und
einem Luer-Verbindungsanschluss (160).

7. Intravenöses Abgabesystem (100) gemäß einem der Ansprüche 1 bis 6, wobei die Vielzahl von Komponenten weiterhin eine Vielzahl von Außenflächen umfasst, wobei sich die eine oder die mehreren Antikoagulansbeschichtungen (300) auf im Wesentlichen allen der Vielzahl von Innenflächen und auf einer ersten Außenfläche der Vielzahl von Außenflächen befinden.

8. Verfahren zur Herstellung eines intravenösen Abgabesystems (100), wobei das Verfahren umfasst:
Bereitstellen einer Vielzahl von Komponenten des intravenösen Abgabesystems (100), so dass die Vielzahl von Komponenten eine Vielzahl von Innenflächen umfassen, welche zusammen einen Fluidweg definieren, durch den ein Fluid in einen Körper eines Patienten fließt;
Herstellen einer Antikoagulanslösung;
Einwirkenlassen der Antikoagulanslösung auf wenigstens eine erste Innenfläche der Vielzahl von Innenflächen unter Bildung von Antikoagulansbeschichtungen (300), die die Blutgerinnselbildung in dem Fluidweg einschränken; und
Bewirken, dass die Antikoagulansbeschichtungen (300) an wenigstens der ersten Innenfläche haften;
wobei das Verfahren **dadurch gekennzeichnet ist, dass** das Herstellen einer Antikoagulanslösung das Auflösen eines Triblock-Copolymers in Wasser oder anderen Lösungen umfasst.

9. Verfahren gemäß Anspruch 8, wobei das Triblock-Copolymer aus der Gruppe ausgewählt ist, die aus PEO-PPO-PEO und PEO-PBD-PEO besteht.

10. Verfahren gemäß Anspruch 9, wobei das Herstellen der Antikoagulanslösung weiterhin das Auflösen von niedermolekularem Heparin oder einem anderen gerinnungshemmenden Wirkstoffmolekül in dem Wasser oder der anderen geeigneten Lösung umfasst.

11. Verfahren gemäß einem der Ansprüche 8 bis 10, wobei das Bewirken, dass die Antikoagulansbeschichtungen (300) an wenigstens der ersten Innenfläche haften, das Ausbilden einer kovalenten Bindung zwischen den Antikoagulansbeschichtungen (300) und der ersten Innenfläche umfasst,
wobei vorzugsweise das Ausbilden einer kovalenten Bindung zwischen der Antikoagulansbeschichtung (300) und der ersten Innenfläche das Anwenden von Strahlung auf die eine oder die mehreren Antikoagulansbeschichtungen (300) und die erste Innenfläche umfasst, um die Bildung der kovalenten Bindung zu induzieren;
wobei besonders bevorzugt das Anwenden von Strahlung auf die eine oder die mehreren Antikoagulansbeschichtungen (300) und die erste Innenfläche das Anwenden einer Auswahl aus der Gruppe, die aus Gammastrahlung, UV-Strahlung und Elektronenstrahlung auf die eine oder die mehreren Antikoagulansbeschichtungen (300) und die erste Innenfläche umfasst.

12. Verfahren gemäß einem der Ansprüche 8 bis 11, wobei das Einwirkenlassen der Antikoagulanslösung auf wenigstens eine erste Innenfläche der Vielzahl von Innenflächen das Befestigen der einen oder der mehreren Antikoagulansbeschichtungen (300) auf der ersten Innenfläche als eine oder mehrere PEO-Bürstenschichten, die jeweils eine Dicke von weniger als 20 Nanometer aufweisen, umfasst.

13. Verfahren gemäß Anspruch 12, wobei das Einwirkenlassen der Antikoagulanslösung auf wenigstens eine erste Innenfläche der Vielzahl von Innenflächen das Einwirkenlassen der Antikoagulanslösung auf eine erste Komponente der Vielzahl von Komponenten umfasst, die aus der Gruppe ausgewählt sind, die aus Folgenden besteht:
einer Katheterschlauchspitze (110);
einem Katheterschlauch (120);
einem Katheteradapter (130);
integrierten Verlängerungsschläuchen (140); und
einem Luer-Verbindungsanschluss (160).

14. Verfahren gemäß einem der Ansprüche 8 bis 13, wobei das Einwirkenlassen der Antikoagulanslösung auf wenigstens eine erste Innenfläche der Vielzahl von Innenflächen das Einwirkenlassen der Antikoagulanslösung auf im Wesentlichen alle der Vielzahl von Innenflächen umfasst; und
wobei die Vielzahl von Komponenten weiterhin eine Vielzahl von Außenflächen umfasst, wobei das Verfahren weiterhin das Einwirkenlassen der Antikoagulanslösung auf eine erste Außenfläche der Vielzahl von Außenflächen umfasst.

15. Verfahren gemäß Anspruch 8, wobei die Vielzahl von Komponenten wenigstens einen Katheterschlauch, einen Adapter und integrierte Schläuche umfasst;
Einwirkenlassen der Antikoagulanslösung auf wenigstens eine Teilmenge von Innenflächen der Vielzahl von Innenflächen unter Bildung von Antikoagulansbeschichtungen (300) auf der Teilmenge von Innenflächen, die die Blutgerinnselbildung in dem Fluidweg einschränken, wobei sich die Teilmenge von Innenflächen auf wenigstens dem Katheterschlauch, dem Adapter und den integrierten Schläuchen befindet; und
Anwenden von Strahlung auf die Antikoagulansbeschichtungen (300) und die Teilmenge von Innenflächen unter Bildung von kovalenten Bindungen zwischen den Antikoagulansbeschichtungen (300) und die Teilmenge von Innenflächen.

## Revendications

1. Système d'administration intraveineuse (100) comprenant :
une pluralité de composants comprenant une pluralité de surfaces intérieures qui coopèrent pour définir une voie de passage de fluide à travers laquelle le fluide s'écoule dans le corps d'un patient ; et
un ou plusieurs revêtement(s) anticoagulant(s) (300) sur au moins une première surface intérieure de la pluralité de surfaces intérieures,
dans lequel le ou les plusieurs revêtement(s) anticoagulant(s) (300) limite/limitent la formation de caillots sanguins dans la voie de passage de fluide,
dans lequel le système d'administration intraveineuse (100) est **caractérisé en ce que** le ou les plusieurs revêtement(s) anticoagulant(s) (300) comprend/comprennent un copolymère tribloc.

2. Système d'administration intraveineuse (100) de la revendication 1, dans lequel le copolymère tribloc est choisi dans le groupe constitué de PEO-PPO-PEO et PEO-PBD-PEO.

3. Système d'administration intraveineuse (100) de la revendication 1, dans lequel le ou les plusieurs revêtement(s) anticoagulant(s) est/sont lié(s) par covalence à la première surface intérieure.

4. Système d'administration intraveineuse (100) de l'une quelconque des revendications 1 à 3, dans lequel le ou les plusieurs revêtement(s) anticoagulant(s) (300) est/sont fixé(s) à la première surface intérieure sous forme d'une ou de plusieurs couche(s) de brosse PEO, chacune ayant une épaisseur inférieure à 20 nanomètres.

5. Système d'administration intraveineuse (100) de la revendication 1, dans lequel le ou les plusieurs revêtement(s) anticoagulant(s) (300) comprend/comprennent en outre de l'héparine de faible poids moléculaire.

6. Système d'administration intraveineuse (100) de l'une quelconque des revendications 1 à 5, dans lequel la première surface intérieure se trouve sur un premier composant de la pluralité de composants, dans lequel le premier composant est choisi dans le groupe constitué par :
une pointe de tubulure de cathéter (110) ;
une tubulure de cathéter (120) ;
un adaptateur de cathéter (130) ;
une tubulure d'extension intégrée (140) ; et
un orifice de raccord Luer (160).

7. Système d'administration intraveineuse (100) de l'une quelconque des revendications 1 à 6, dans lequel la pluralité de composants comprennent en outre une pluralité de surfaces extérieures, dans lequel le ou les plusieurs revêtement(s) anticoagulant(s) (300) se trouve/trouvent essentiellement sur la totalité de la pluralité de surfaces intérieures, et sur une première surface extérieure de la pluralité de surfaces extérieures.

8. Procédé de fabrication d'un système d'administration intraveineuse (100), le procédé comprenant le fait :
de fournir une pluralité de composants du système d'administration intraveineuse (100) de sorte que la pluralité de composants comprennent une pluralité de surfaces intérieures qui coopèrent pour définir une voie de passage de fluide à travers laquelle le fluide s'écoule dans le corps d'un patient ;
de préparer une solution anticoagulante :
d'exposer au moins une première surface intérieure de la pluralité de surfaces intérieures à la solution anticoagulante pour former des revêtements anticoagulants (300) qui limitent la formation de caillots sanguins dans la voie de passage de fluide ; et
d'amener les revêtements anticoagulants (300) à adhérer à au moins la première surface intérieure ;
dans lequel le procédé est **caractérisé en ce que** la préparation d'une solution anticoagulante comporte la dissolution d'un copolymère tribloc dans de l'eau ou d'autres solutions.

9. Procédé de la revendication 8, dans lequel le copolymère tribloc est choisi dans le groupe constitué de PEO-PPO-PEO et PEO-PBD-PEO.

10. Procédé de la revendication 9, dans lequel la préparation de la solution anticoagulante comprend en outre la dissolution de l'héparine de faible poids moléculaire ou d'une autre molécule de médicament anticoagulant dans de l'eau ou une autre solution appropriée.

11. Procédé de l'une quelconque des revendications 8 à 10, dans lequel le fait d'amener les revêtements anticoagulants (300) à adhérer à au moins la première surface intérieure comprend la formation d'une liaison covalente entre les revêtements anticoagulants (300) et la première surface intérieure, de préférence
dans lequel la formation d'une liaison covalente entre le revêtement anticoagulant (300) et la première surface intérieure comprend l'application d'un rayonnement au ou aux plusieurs revêtement(s) anticoagulant(s) (300) et à la première surface intérieure pour induire la formation de la liaison covalente, plus préférablement
dans lequel l'application d'un rayonnement au ou aux plusieurs revêtement(s) anticoagulant(s) (300) et à la première surface intérieure comprend l'application d'une sélection dans le groupe constitué par l'irradiation gamma, l'irradiation aux ultraviolets et l'irradiation par faisceau d'électrons au ou aux plusieurs revêtement(s) anticoagulant(s) (300) et à la première surface intérieure.

12. Procédé de l'une quelconque des revendications 8 à 11, dans lequel l'exposition d'au moins une première surface intérieure de la pluralité de surfaces intérieures à la solution anticoagulante comprend la fixation du ou des plusieurs revêtement(s) anticoagulant(s) (300) à la première surface intérieure sous forme d'une ou de plusieurs couche(s) de brosse PEO, chacune ayant une épaisseur inférieure à 20 nanomètres.

13. Procédé de la revendication 12, dans lequel l'exposition d'au moins une première surface intérieure de la pluralité de surfaces intérieures à la solution anticoagulante comprend l'exposition, à la solution anticoagulante, d'un premier composant de la pluralité de composants choisi dans le groupe constitué par :
une pointe de tubulure de cathéter (110) ;
une tubulure de cathéter (120) ;
un adaptateur de cathéter (130) ;
une tubulure d'extension intégrée (140) ; et
un orifice de raccord Luer (160).

14. Procédé de l'une quelconque des revendications 8 à 13, dans lequel l'exposition d'au moins une première surface intérieure de la pluralité de surfaces intérieures à la solution anticoagulante comprend le fait d'exposer essentiellement la totalité de la pluralité de surfaces intérieures à la solution anticoagulante ; et
dans lequel la pluralité de composants comprennent en outre une pluralité de surfaces extérieures, le procédé comprenant en outre l'exposition d'une première surface extérieure de la pluralité de surfaces extérieures à la solution anticoagulante.

15. Procédé de la revendication 8, dans lequel la pluralité de composants comprend au moins une tubulure de cathéter, un adaptateur et une tubulure intégrée ;
l'exposition d'au moins un sous-ensemble de surfaces intérieures de la pluralité de surfaces intérieures à la solution anticoagulante pour former des revêtements anticoagulants (300) sur le sous-ensemble de surfaces intérieures qui limitent la formation de caillots sanguins dans la voie de passage de fluide, où le sous-ensemble de surfaces intérieures se trouve sur au moins la tubulure de cathéter, l'adaptateur et la tubulure intégrée ; et
l'application d'un rayonnement aux revêtements anticoagulants (300) et au sous-ensemble de surfaces intérieures pour former des liaisons covalentes entre les revêtements anticoagulants (300) et le sous-ensemble de surfaces intérieures.
